# EUROPEAN PATENT SPECIFICATION

(11) **EP 0 969 822 B1**
(45) Date of publication and mention of the grant of the patent: **26.03.2003**
(21) Application number: 97953486.4
(22) Date of filing: 29.12.1997
(51) Int. Cl.: A61B 10/00

(54) **DETERMINATION OF THE PROPENSITY OF AN ORGAN OR TISSUE FOR CANCER BY DETERMINING OF ITS IMPRINTING PATTERN**
BESTIMMUNG DER KREBSANFÄLLIGKEIT EINES ORGANS ODER GEWEBES DURCH BESTIMMUNG SEINER ZELLPRÄGUNG
DETERMINATION DU RISQUE DE CANCER D'UN ORGANE OU TISSUE EN ETABLISSANT SON EMPREINTE CELLULAIRE

(30) Priority: 30.12.1996 US 34095 P
(43) Date of publication of application: 12.01.2000
(73) Proprietor: THE JOHNS HOPKINS UNIVERSITY SCHOOL OF MEDICINE, Baltimore, MD 21205 (US)
(72) Inventor: FEINBERG, Andrew P., Lutherville, MD 21093 (US)
(74) Representative: Koch, Gustave
(86) International application number: US9723991
(87) International publication number: WO98029108

(56) References cited:
- EP-A- 0 627 436
- WO-A-92/15602
- US-A- 4 497 803
- US-A- 4 690 918
- US-A- 5 356 903
- J.M. BARLETTA ET AL.: "Reversal of Loss of Imprinting in Tumor Cells by 5-Aza-2'-deoxycytidine" HTTP://AACR.EDOC.COM/CR/V57N1/CH000048.HTM L, XP002066097 & "Cancer Research; Instructions for Authors" HTTP://WWW.AACR.ORG/CANINST.HTML, pages 1-19, & J.M.BARLETTA ET AL.: "Reversal of Loss of Imprinting in Tumor Cells by 5-Aza-2'-deoxycytidine" CANCER RESEARCH, vol. 57, no. 1, 1 January 1997, pages 48-50,
- P. EVERSOLE-CIRE ET AL.: "Activation of an Imprinted Igf 2 Gene in Mouse Somatic Cell Cultures" MOL.CELL.BIOL., vol. 13, no. 8, August 1993, pages 4928-4838, XP002066098
- HTTP://CPMCNET.COLUMBIA.EDU/NEWS/JOURNAL/A RCHIVES/JOUR_V15N1_0005.HTML, XP002070304 & "Wilms' Tumor Gene Research" P&S JOURNAL, vol. 15, no. 1, 1995,
- J. WEBER ET AL.: "Expression of the MAGE-1 Tumor Antigen Is Up-Regulated by the Demethylating Agent 5-Aza-2'-Deoxycytidine" CANCER RES., vol. 54, no. 7, 1 April 1994, pages 1766-1771, XP002066099
- S.W.LEE ET AL.: "Down-regulation of a member of the S100 gene family in mammary carcinoma cells and reexpression by azadeoxycytidine treatment" PROC.NATL.ACAD.SCI. USA, vol. 89, no. 6, March 1992, pages 2504-2508, XP002066100
- A.T.FERGUSON ET AL.: "Demethylation of the Estrogen Receptor Gene in Estrogen Receptor-negative Breast Cancer Cells Can Reactivate Estrogen Receptor Gene Expression" CANCER RES., vol. 55, no. 11, 1 June 1995, pages 2279-2283, XP002066101
- M.CARAGLIA ET AL.: "5-Aza-2'-deoxycytidine induces growth inhibition and upregulation of epidermal growth factor receptor on human epithelial cancer cells" ANN.ONCOL., vol. 5, no. 3, March 1994, pages 269-276, XP002066102
- W.-Y. CHUNG ET AL.: "Chromosome 11p15.5 regional imprinting: comparative analysis of KIP2 and H19 in human tissues and Wilm's tumors" HUM.MOL.GENET., vol. 5, no. 8, August 1996, pages 1101-1108, XP002066103
- B. TYCKO: "DNA methylation in genomic imprinting" MUTATION RESEARCH, vol. 386, no. 2, 1997, pages 131-140, XP002070305
- L.A.MICHALOWSKY ET AL.: "DNA Methylation and Differentiation" ENVIRON.HEALTH PERSPECT., vol. 80, March 1989, pages 189-197, XP002066104
- J.-F. HU ET AL.: "Promotor-specific Modulation of Insulin-like Growth Factor II Genomic Imprinting by Inhibitors of DNA Methylation" J.BIOL.CHEM., vol. 271, no. 30, 26 July 1996, pages 18253-18262, XP002066105
- A.H.BEGGS ET AL.: "Reactivation of X-Linked Genes in Human Fibroblasts Transformed by Origin-Defective SV40" SOMAT.CELL MOL. GENET., vol. 12, no. 6, November 1986, pages 585-594, XP002066106
- S.M. TAYLOR: "5-Aza-2'-Deoxycytidine: Cell Differentiation and DNA Methylation" LEUKEMIA, vol. 7, no. Suppl. 1, May 1993, pages 3-8, XP002066107
- S. RAINIER ET AL.: "Capture and characterization of 5-aza-2'-deoxycytidine-treated C3H/10T1/2 cells prior to transformation" PROC.NATL.ACAD.SCI. USA, vol. 85, no. 17, September 1988, pages 6384-6388, XP002066108

## Description

### FEDERALLY SPONSORED RESEARCH OR DEVELOPMENT

This invention was made with Government support under grants from the National Institutes of Health. The Government has certain rights to this invention.

### FIELD OF THE INVENTION

The present invention relates to a process of diagnosing the propensity of a mammal to develop cancer in a target organ or tissue.

### BACKGROUND OF THE INVENTION

Genomic imprinting is an epigenetic modification of a specific parental chromosome in the gamete or zygote that leads to monoallelic or differential expression of the two alleles of a gene in somatic cells of the offspring. Imprinting affects various essential cellular and developmental processes, including intercellular signaling, RNA processing, cell cycle control, and promotion or inhibition of cellular division and growth.

The first deduction of imprinting at the single gene level involved a transgenic C-myc gene which showed dependance of its expression on paternal inheritance. The silent maternally inherited copy was methylated (Swain *et al*. (1987) *Cell* 50:719-727).

Disruption of the insulin-like growth factor II (IGF2) gene in knockout experiments showed that IGF2 is imprinted and expressed normally only from the paternal allele, and that IGF2 is biparentally expressed in two neural tissues, the choroid plexus and the leptomeninges (DeChiara *et al.* (1991) *Cell* 64:849-859). These milestone studies showed that genomic imprinting affects normal endogenous genes and that imprinting shows tissue-specific regulation.

Direct approaches developed to identify novel imprinted genes include: positional cloning which identifies imprinted genes near other known imprinted genes (Barlow *et al.* (1991) *Nature* 349:84-87); comparing gene expression in parthenogenetic embryos to that of normal embryos (Kuroiwa *et al.* (1996) *Nat. Genet* 12:186-190); and restriction landmark genome scanning (Nagai *et al.* (1995) *Biochem. Bionhys. Res. Commun. 213:258-265).* The last approach comprises analysis of clonality in tumors by assessing DNA methylation near a heterozygous polymorphic site (Vogelstein *et al.* (1985) *Science* 227:642-645).

Abnormalities of a single gene can affect imprinting of a proximate genomic region and disrupt multiple disease-causing genes, the phenotype depending upon the parental origin of the mutated gene. Two examples of imprinted human disease loci in close proximity are on chromosome 15. Disrupted imprinting of these loci is one of the causes of Prader-Willi syndrome (PWS) and Angelman syndrome (AS) which involve mental retardation. PWS also causes obesity, and AS involves gross motor disturbances. Each disorder can be caused by parental-origin specific uniparental disomy (Nicholls *et al.* (1989) *Nature* 342:281-285; Knoll *et* al. (1990) *Am. J. Hum. Genet.* 47:149-155). or chromosomal deletions (Knoll *et al.* (1989) *Am. J. Hum. Genet.* 47:149-155; Mattel *et al.* (1984) *Hum. Genet.* 66:313-334). The AS gene has not been isolated to date. PWS appears to be due to mutations or deletions in the small nuclear ribonucleoprotein polypeptide N (SNRPN) gene (reviewed in Nicholls *et al*. (1993) *Curr. Opin. Genet. Dev*. 3:445-446). A mutation affecting splicing of an untranslated upstream exon of SNRPN can also lead to AS, as well as abnormal imprinting of other loci (Dittrich *et al.* (1996) *Nat. Genet.* 14:163-170).

It is likely that many more additional imprinted human disease loci will be identified, because UPD for specific chromosomes is often associated with multiple congenital anomalies (Ledbetrer, D.H. and Engel, E. (1995) *Hum. Mol. Genet.* 4:1757-1764). Chromosomes that likely show this phenomenon include 2, 6, 7, 11,14, 15, 16, 20, and X (Ledbetter, D.H. and Engel, E. (1995) *Hum. Mol. Genet.* 4:1757-1764).

Implication of genomic imprinting in cancer came from investigations of the two embryonic tumors, hydatidiform mole and complete ovarian teratoma, showing that not only 46 chromosomes are required to create a normal embryo, but also a balance of maternal and paternal chromosomes. A relative imbalance leads to neoplastic growth, and the type of neoplasm depends upon whether there is a maternal or paternal genetic excess.

Another tumor apparently associated with imprinting is familial paraganglioma or glomus tumor. In all cases, the transmitting parent is the father (Van der Mey *et al*. (1989) *Lancet* 2:1291-1294). The gene has recently been localized to 11q22.3-q23 (Heutink *et al.* (1994) *Eur. J. Hum. Genet* 2:148-158).

Loss of heterozygosity (LOH) in the childhood Wilms tumor occurs on chromosome 11 and the specifically involved region is 11p15 (Reeve *et al*. (1989) *Mol. Cell. Biol.* 9:1799-1803). Schroeder *et al*. (1987) *Am. J. Hum. Genet.* 40: 413-420, noted that in five of five cases of LOH, it was the maternal allele that was lost. This observation has been extended to other tumors of embryonal origin such as hepatoblastoma and rhabdomyosarcoma (Scrable *et al.* (1989) *Proc. Natl. Acad. Sci. USA* 86:7480-7484).

The N-myc gene on chromosome 2 shows preferential amplification of the paternal allele in neuroblastoma (Cheng *et al.* (1993) *Nature Genet.* 4:187-190). Advanced neuroblastoma tumors, showing N-myc amplification, also show preferential LOH of maternal chromosome 1, whereas earlier stage tumors without N-myc amplification do not (Caron *et al*. (1995) *Hum. Mol. Genet* 4:535-539). Thus, genetic disturbances involving imprinted genes in a given type of cancer may involve multiple chromosomes concurrently. Naumova *et al*. (1994) *Am. J. Hum. Genet.* 54:274-281, found transmission ratio distortion, concordance of 13q loss and isochromosome 6 of the same parental origin in retinoblastoma, again consistent with a mechanism of generalized disturbance of imprinting in embryogenesis leading to increased cancer risk.

Beckwith-Wiedemann syndrome (BWS) is a disorder of prenatal overgrowth and cancer, transmitted as an autosomal dominant trait, or arising sporadically. A clue to a role for genomic imprinting is increased disease penetrance when BWS is inherited from the mother (Viljoen, D. and Ramesar, R. (1992) *J. Med. Genet* 29:221-225). The tumors of children show preferential loss of maternal 11p15 (Schroeder *et al.* (1987) *Am. J. Hum. Genet* 40:413-420; Scrable *et al.* (1989) *Proc. Natl. Acad. Sci. USA* 86:7480-7484), suggesting that an imprinted locus could cause BWS and also be involved in sporadically occurring tumors. Genetic linkage analysis showed that BWS localizes to 11p15, consistent with this idea of generalized disruption, and not to 11p13, to which the WT1 gene had been localized (Ping *et al.* (1989) *Am. J. Hum. Genet.* 44:720-723; Koufos *et al.* (1989) *Am. J. Hum. Genet.* 44:711-719).

More direct evidence for genomic imprinting of 11p15 in BWS came from studies showing that some patients with BWS have paternal UPD, involving a region extending from the β-globin locus to the RAS gene (Henry *et al.* (1991) *Nature* 35:609-610). While the large 10 Mb region does not provide precise localization of an imprinted gene, it provides a foundation for later studies of imprinted loci on this chromosome.

Examination of RNA from Wilms tumor (WT) led to a discovery that not one but both IGF2 alleles were expressed in 70% of Wilms tumors (Rainier *et al*. (1993) *Nature* 362:747-749; Ohlsson *et al*. (1993) *Nature Genet* 4:94-97). In addition, in 30% of cases, both alleles of H19 were expressed (Rainier *et al*. (1993) *Nature* 362:747-749). In contrast, examination of RNA from normal tissue show normal imprinting with the expression of one allele of IGF2 and H19. This was the first evidence of imprinting in humans. The term for this novel genetic alteration is loss of imprinting (LOI) (Rainier *et al*. (1993) *Nature* 362:747-749) which simply means loss of preferential parental origin-specific gene expression can involve either abnormal expression of the normally silent allele, leading to biallelic expression, or silencing of the normally expressed allele, leading to epigenetic silencing of the locus (Rainier *et al.* (1993) *Nature* 362:747-749; Feinberg (1993) *Nature Genet.* 4:110-113; Feinberg *et al.* (1995) *J. Natl. Cancer Inst. Monographs* 17:21-26). Thus, abnormal imprinting in cancer can lead to activation of normally silent alleles of growth-promoting genes (Rainier *et al.* (1993) *Nature* 362:747-749); (Feinberg (1993) *Nature Genet* 4:110-113); (Feinberg *et al.* (1995) *J. Natl. Cancer Inst. Monographs* 17:21-26).

Subsequently, LOI has been implicated in various tumor types. At first, LOI was found in other childhood tumors, such as hepatoblastoma (Rainier *et al*. (1995) *Cancer* *Res.* 55:1836-1838); (Li *et al.* (1995) *Oncogene* 11:221-229). rhabdomyosarcoma (Zhan *et al.* (1994) *J. Clin. Invest.* 94:445-448), and Ewing's sarcoma (Zhan *et al*. (1995a) *Oncogene* 11:2503-2507). LOI of IGF2 and H19 have also now been found in many adult tumors, including uterine (Vu *et al.* (1995) J. Clin. Endocrinol. Metab. 80:1670-1676, cervical (Doucrasy *et al.* (1996) *Oncogene* 12:423-430), esophageal (Hibi *et al*. (1996) *Cancer Res.* 56:480-482), prostate (Jarrard *et al.* (1995) *Clin. Cancer Res.* 1:1471-1478), lung cancer (Kondo *et al.* (1995) *Oncogene* 10:1193-1198), choriocarcinoma (Hashimoto *et al.* (1995) *Nat. Genet.* 9:109-110), germ cell tumors (Van Gurp *et al.* (1994) *J. Natl. Cancer Inst.* 86:1070-1075) BWS (Steenman *et al.* (1994) *Nature Genet.* 7:433-439); Weksberg *et al.* (1993) *Nature Genet.* 5:143-150) and Wilms tumor (Ogawa *et al.* (1993) *Nature Genet* 5:408-412). Thus, LOI is one of the most common genetic alterations in human cancer.

Another imprinted gene in 11p15 is p57^{KIP2}, a cyclin-dependent kinase (CDK) inhibitor similar in its CDK inhibitory domain to p21^{WAFI/Cip1}, a target of p53 (Matsuoka *et al*. (1995) *Genes Dev.* 9:640-662); Lee *et al*. (1995) *Genes Dev.* 9:639-649). The gene was localized within 40 kb of a group of BWS balanced germline chromosomal rearrangement breakpoints, in contrast to IGF2 and H19, which are located telomeric to those breakpoints (Hoovers *et al.* (1995) *Proc. Natl. Acad. Sci. USA* 92:12456-12460). Human p57^{KIP2} was found to be imprinted with preferential expression from the maternal allele (Matsuoka *et al.* (1996) *Proc. Natl. Acad. Sci. USA* 93:3026-3030). p57^{KIP2} also shows abnormal imprinting and epigenetic silencing in some tumors and BWS patients (Thompson *et al.* (1996) *Cancer Research* 56:5723-5727).

Cytosine DNA methylation is catalyzed by the enzyme cytosine (DNA-5)-methyltransferase (hereinafter, DNA methyltransferase). DNA methylation occurs almost exclusively at CpG dinucleotides (reviewed in Cedar *et al.* (1990) *Biochem. Biophys. Acta* 1049:1-8). The pattern of DNA methylation is heritable by somatic cells and maintained, after DNA replication, by DNA methyltransferase which has a 100-fold greater affinity for hemimethylated DNA (i.e., parent strand methylated, daughter strand unmethylated) than for unmethylated DNA (Cedar *et al.* (1990) *Biochem. Biophys. Acta* 1049:1-8). However, developing cells of the gamete and embryo undergo dramatic shifts in DNA methylation by both loss of methylation and de novo methylation (Cedar *et al.* (1990) *Biochem. Biophys*. *Acta* 1049:1-8). The mechanism establishing the original pattern of DNA methylation is unknown.

Two classes of cytosine DNA methylation exist in the genome. The first, generally associated with gene silencing, occurs on genes that show tissue-specific expression. Since this type occurs both before and after the changes in gene expression, it is not necessarily the cause of altered gene expression during development; but rather may help to "lock in" a given pattern of gene expression (Cedar *et al.* (1990) *Biochem. Biophys. Acta* 1049:1-8); Riggs, A.D. (1989) *Cell Biophys.* 15:1-13). The second class involves CpG islands which are almost always unmethylated in normal cells, and are usually within the promoter or first exon of housekeeping genes (reviewed in Bird, A.P. (1986) *Nature* 321:209-213). However, an important exception is CpG islands on the inactive X-chromosome, which are methylated. Thus, CpG island methylation, unlike non-CpG island methylation, is thought to be involved in epigenetic silencing and/or marking of the inactive X chromosome (reviewed in Riggs, A.D. and Pfeifer, G.P. (1992) *Trends Genet.* 8:169-174.

Several lines of evidence provide a role for DNA methylation in the control of genomic imprinting. First, some imprinted genes in mice, such as H19, show parental origin-specific, tissue-independent methylation of CpG islands (Ferguson-Smith *et al.* (1993) *Nature* 362:751-755); Bartolomei *et al.* (1993) *Genes Develop.* 7:1663-1673. This methylation represents imprinting on the paternal chromosome and is not secondary to changes in gene expression. Second, knockout mice deficient in DNA methyltransferase, and exhibiting widespread genomic hypomethylation, do not show allele-specific methylation of the H19 CpG island and exhibit biallelic expression of H19 and loss of expression of IGF2 (Li *et al*. (1993) *Nature* 366:362-365). Similar parental origin-specific methylation has also been observed for a CpG island in the first intron of the maternally inherited, expressed allele of the IGF2 receptor gene (IGF2R) (Stoger *et al*. (1993) *Cell* 73:61-71). Methyltransferase-deficient knockout mice show loss of methylation of IGF2R and epigenetic silencing of the gene (Li *et al.* (1993) *Nature* 366:362-365).

Widespread alterations in DNA methylation in human tumors were discovered 15 years ago (Feinberg, A.P. (1983) *Nature Genet.* 4:110-113) and remain the most commonly found alteration in human cancers. These alterations are ubiquitous to both benign and malignant neoplasms (Goelz *et al*. (1985) *Science* 228:187-190). The precise role of these changes remain unclear; but, both decreased and increased methylation have been found at specific sites in tumors, with an overall decrease in quantitative DNA methylation (Feinberg *et al*. (1988) *Cancer Res.* 48:1159-1161; Feinberg, A.P. (1988) *Prog. Clin. Biol. Res.* 279:309-317; Jones *et al.* (1990) *Adv. Cancer Res.* 54:1-23).

In humans, as in mice, the paternal allele of a CpG island in the H19 gene and its promoter is normally methylated, and the maternal allele is unmethylated (Steenman *et al.,* (1994) *Nature Genet* 7:433-439; Ferguson-Smith *et al.* (1993) *Nature* 362:751-755; Bartolomei *et al.* (1993) *Genes Develop.* 7:1663-1673). Because tumors with LOI of IGF2 showed reduced expression of H19, the methylation pattern of H19 has been examined in tumors with LOI. In all cases showing LOI of IGF2, the H19 promoter exhibits 90-100% methylation at the sites normally unmethylated on the maternally inherited allele (Steenman *et al.* (1994) *Nature* 7:433-439; Moulton *et al*. (1994) *Nature Genet.* 7:440-447). Thus, the maternal allele has acquired a paternal pattern of methylation, consistent with observed expression of IGF2 on the same maternally derived chromosome in these tumors. In contrast, tumors without LOI of IGF2 show no change in the methylation of H19, indicating that these changes are related to abnormal imprinting and not malignancy per se (Steenman *et al.* (1994) *Nature Genet* 7:433-439; Moulton *et al.* (1994) *Nature Genet.* 7:440-447). The same alterations in methylation of the maternal allele of H19 are found in BWS patients with LOI of IGF2 (Steenman *et al.* (1994) *Nature Genet.* 7:433-439; Reik *et al.* (1994) *Hum. Mol. Genet.* 3:1297-1301; Reik *et al.* (1995) *Hum. Mol. Genet.* 4:2379-2385).

A second potential mechanism of LOI may involve disruption of an imprinting control center on chromosome 11, similar to that recently described for the BWS/AS region of chromosome 15 (Dittrich *et al,* (1996) *Nat. Genet.* 14:163-170). A cluster of five BWS balanced germline chromosomal rearrangement breakpoints lies between p57^{KIP2} on the centromeric side, and IGF2 and H19 on the telomeric side (Hoovers *et al.* (1995) *Proc. Natl. Acad. Sci. USA* 92:12456-12460). Thus, disruption of a gene spanning this region could cause abnormal imprinting, as well as BWS and/or cancer, at least when inherited through the germline.

Another potential mechanism for LOI involves loss of trans-acting factors which may establish and maintain a normal pattern of genomic imprinting once such a pattern is established in the germline. Trans-acting modifiers of imprinting are likely to exist, since imprinting of transgenes is host strain-dependent (Sapienza *et al.* (1990) *Develop.* 107:165-168; Allen *et al.* (1990) *Cell* 61:853-361). Such genes might thus act as tumor suppressor genes in humans and other species.

Yet another potential mechanism of imprinting that might be disrupted in cancer involves histone deacetylation, which is linked to X-inactivation in mammals (reviewed in Wolffe, A.P. (1994) *Develop. Genet.* 15:463-470) and to telomere silencing in yeast (Thompson *et al.* (1994) *Nature* 369:245-247). Genes for both histone acetylase and histone deacetylase have recently been isolated (Brownell *et al.* (1996) *Cell* 84:843-851; Taunton *et al.* (1996) *Science* 272:408-411). In addition, telomere silencing in yeast also involves the action of specific genes, e.g., SIR1-SIR4, some of which have homologues in mammals (Brachmann *et al.* (1995) *Genes Develop.* 9:2888-2902). Similarly, some examples of gene silencing in mammals may resemble position-effect variation in Drosophila, a form of position-dependent epigenetic silencing (Walters *et al.* (1996) *Genes Develop.* 10:185-195). Finally, imprinted loci on maternal and paternal chromosomes may interact during DNA replication. Chromosomal regions harboring imprinted genes show replication and timing asynchrony (Kitsberg *et al.* (1993) *Nature* 364:459-463). Furthermore, the two parental homologues of some imprinted genes show nonrandom proximity in late S-phase (LaSalle, J.M. and Lalande, M. (1996) *Science 272:725-728),* suggesting some form of chromosomal cross-talk, as has been observed for epigenetic silencing in Drosophila (Tatof, K.D. and Henikoff, S. (1991) *Cell 65:201-203).*

The human IGF2 and H19 genes are normally imprinted, *i.e.,* show preferential expression of a specific parental allele (Rainier *et al.* (1993) *Nature* 362:747-749; Zhang, Y. and Tycko, B. (1992) *Nat. Genet.* 1:40-44; Giannoukakis *et al*. (1993) *Nat. Genet.* 4:98-101; Ohisson *et al*. (1993) *Nature Genet.* 4:94-97; Ogawa *et al*. (1993b) *Nature* 362:749-751). Furthermore, as discussed above, some tumors undergo loss of imprinting (LOI) in cancer, with one or more of the following: biallelic expression of IGF2 (Rainier *et al*. (1993) *Nature* 362:747-749; Ogawa *et al*. (1993b) *Nature* 362:749-751), epigenetic silencing of H19 (Steenman *et al*. (1994) *Nature Genet* 7:433-439; Moulton *et al*. (1994) *Nature Genet.* 7:440-447); and/or abnormal expression of the paternal H19 allele (Rainier *et al.* (1993) *Proc. Natl. Acad. Sci. USA* 85:6384-6388), and this observation has been extended to a wide variety of childhood and adult malignancies (Rainier *et al.* (1993) *Proc. Natl. Acad. Sci. USA* 85:6384-6388; Suzuki *et al* (1994) *Nat. Genet.* 6:332-333). Normal imprinting can be maintained in part by allele-specific, tissue-independent methylation of H19, since LOI is associated with abnormal methylation of the normally unmethylated maternal H19 allele (Steenman *et al.* (1994) *Nature Genet.* 7:433-439; Moulton *et al.* (1994) *Nature Genet.* 7:440-447).

Alterations of genomic imprinting have been recently identified in human cancer, appearing commonly in both childhood and adult malignancies. While the art teaches abnormal imprinting and its association with disease states, there is no methods in the art for restoring normal imprinting to such cells. Thus, methods are needed in the art to restore a normal pattern of imprinting to cells.

### SUMMARY OF THE INVENTION

The invention is drawn to methods for determining the propensity of an individual to develop cancer or a malignant cell growth. Such diagnosis involves determining the pattern of imprinting for cells of interest. The determination can be followed by preventive therapies where abnormal imprinting is found.

### BRIEF DESCRIPTION OF THE DRAWINGS

The invention is better understood by reading the following detailed description with reference to the accompanying figures, in which like reference numerals refer to like elements throughout, and in which:

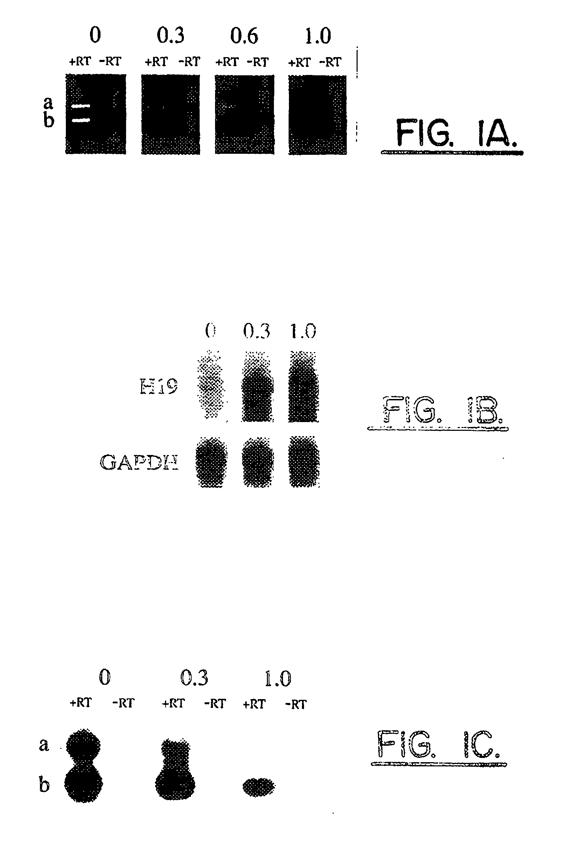

**Figure 1.** Figure 1 depicts the effects of 5-azaCdR on imprinting of tumor cells with LOI. A) Switch to preferential allelic expression of IGF2. RT-PCR was performed on total RNA extracted from JEG-3 cefls after a single 24 hr treatment with 0, 0.3, 0.6, or 1.0 µM 5-azaCdR (indicated). Alternating lanes represent simultaneous experiments with and without reverse transcriptase. PCR products were digested with Apa I. The a and b alleles are 236 and 173 bp, respectively. B) Induction of H19 expression. Expression of U19 in JEG-3 cells after treatment with 5-azaCdR was measured by Northern blot analysis. Blots were rehybridized with GAPDH as a control for loading. C) Switch to monoallelic expression of H19. RT-PCR was performed as in (A) above. PCR products were digested with Alu I to detect allele-specific expression. The a and b alleles are 227 and 100 bp, respectively. Please note that the RT-PCR assay is not quantitative, and the total amounts of RNA in the three sets were not normalized.

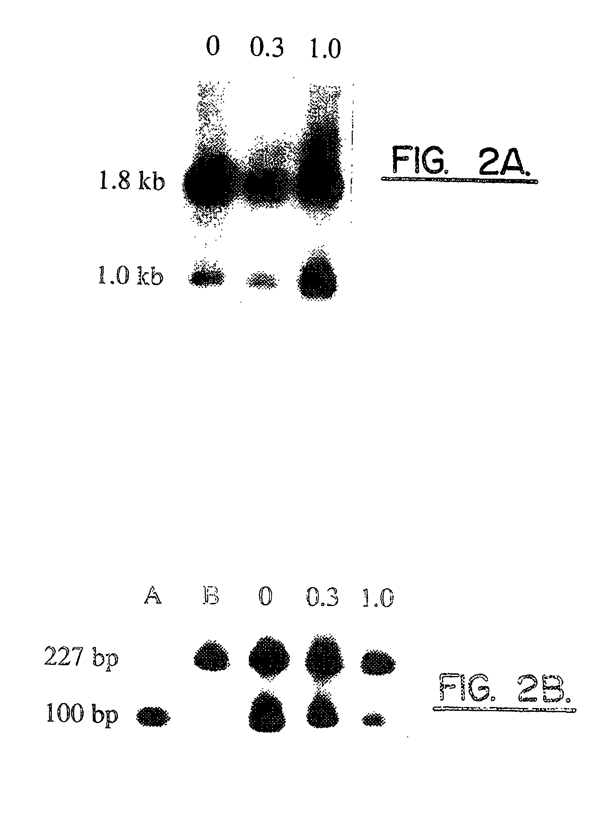

**Figure 2.** Figure 2 depicts the effects of 5-azaCdR on H19 promoter methylation. (A) Decreased methylation of H19 after 5-azaCdR treatment. DNA was extracted from cells treated with 0, 0.3, or 1.0 µM 5-azaCdR, and digested with Pst I and Sma I to assay DNA methylation as descnbed. The 1.8 kb fragment represents methylated DNA and the 1.0 kb fragment unmethylated DNA. (B) Allele specificity of altered methylation. BamH I and Hpa II digested DNA was PCR-amplified and digested with Alu I to detect allele-specific methylation. The A and B alleles (shown) are 227 and 100 bp, respectively.

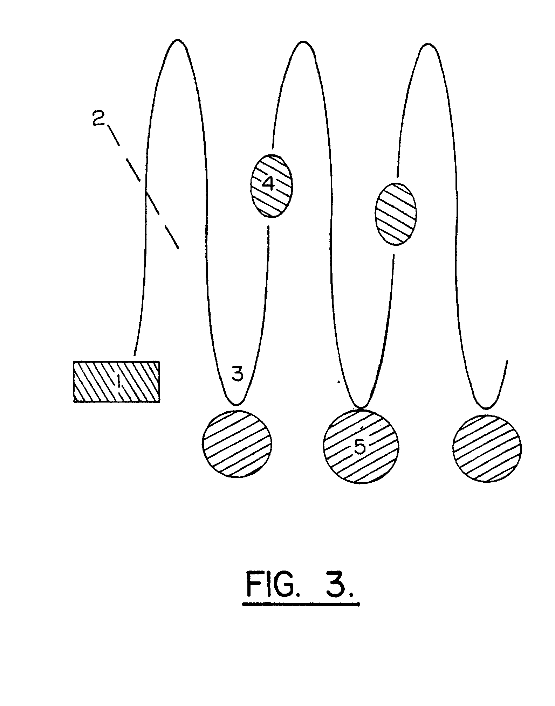

**Figure 3.** Figure 3 depicts a unified model of LOI occurring in some cancers, such as Wilms tumor, which explains both epigenetic silencing of tumor suppressor genes, as well as activation of normally silent alleles of growth-promoting genes. According to this model, LOI involves a switch in the epigenotype of a chromosomal region) for example, in the case of Wilms tumor from maternal to paternal. Thus, IGF2 shows biallelic expression and H19 undergoes epigenetic silencing. However, this model is not meant to be universal. For example, not all tumors show a switch in expression of all three genes, IGF2, H19 and p57^{KIP2}. Hepatoblastoma shows LOI of IGF2, but tumors with biallelic expression of IGF2 do not necessarily undergo epigenetic silencing of H19. In fact, it is recognized that now that the present invention has demonstrated that a normal pattern of imprinting can be restored in cells exhibiting LOI, a number of pathways or mechanisms will become available. Thus, the present invention is not limited to any particular mechanism of restoration of a normal pattern of imprinting.

More specifically, Figure 3 shows an imprint organizing center (red rectangle), exerts a long-range cis-acting influence on IGF2 and other imprinted genes (blue ellipses), via alterations in chromatin structure (represented as DNA loops). This imprint organizing center establishes the imprinting mark as maternal or paternal. This effect is propagated outward during development, similar to the organizing center on the X-chromosome. Imprinting is maintained in part by allele-specific methylation of CpG islands, as well as interactions with trans-acting proteins (green circles). According to this model, loss of imprinting could arise by any of several mechanisms (numbered in the figure): (1) Deletion or mutation in the imprint organizing center itself, which would lead to a failure of parental origin-specific switching in the germline; (2) separation of the imprint organizing center from the imprinted target genes, as seen in BWS germline chromosomal rearrangement cases; (3) abnormal methylation of CpG islands; (4) local mutation of regulatory sequences controlling the target imprinted genes themselves; or (5) loss of or mutations in genes for trans-acting factors that maintain normal imprinting. Figure modified from Feinberg *et al.* (1994) Cambridge University Press, 19:73-292.

### DETAILED DESCRIPTION OF THE INVENTION

Imprinting has been observed in eukaryotic cells of plants and mammals (Yoder and Bestor (1996) *Biol. Chem. 377(10)*: 605-610). In humans and other mammals, normal imprinting underlies several fundamental cellular and developmental processes; thus, abnormal imprinting patterns are implicated in a wide variety of catastrophic human diseases. Restoration of normal imprinting patterns, as provided by the present invention, is useful for diagnosis, prevention and therapy of diseases associated with abnormal imprinting. Imprinting is defined as an epigenetic modification of a specific parental allele of a gene, or the chromosome on which it resides, in the gamete or zygote, leading to differential expression of the two alleles in somatic cells of the offspring.

"Normal pattern of imprinting" means preferential expression of a single parental allele of an imprinted gene and/or preferential methylation of a single parental allele of an imprinted gene. "Loss of imprinting or LOI" means loss of a normal pattern of imprinting, i.e., the loss of preferential expression of a single parental allele of an imprinted gene and/or the loss of methylation of a single parental allele of an imprinted gene. LOI is exhibited by a variety of abnormal expression patterns. Such patterns include but are not limited to: equal expression of both alleles; significant (>5%) expression of the normally silent allele when the normal case is complete silencing of one allele; epigenetic silencing of the normally expressed copy of an imprinted gene; the absence of methylation of both alleles and/or the methylation of both alleles where the normal case is methylation of a single allele.

Thus, "restoration of normal imprinting" means restoring preferential expression of a single parental allele of an imprinted gene. It is recognized that various mechanisms are available for restoring normal imprinting and all such mechanisms are encompassed herein. Such mechanisms include: the relative silencing of one copy of an allele pair where both alleles were expressed at significant-comparative levels prior to treatment; the restoration of preferential expression of the same parental allele that is normally expressed; the preferential expression of a specific allele in a pattern opposite to that seen in normal tissues (allele switching); the re-expression of one copy of an imprinted gene where both copies had been silenced; the restoration of a normal pattern of methylation to allele pairs, i.e., where one allele is methylated and the other allele is not, where abnormal imprinting was exhibited by the absence of methylation on both alleles or alternatively by the methylation of both alleles; and other mechanisms. The importance of each mechanism is that the preferential expression of a single parental allele is restored.

For diseases associated with abnormal imprinting in some instances, the genes exhibiting abnormal imprinting have been elucidated. In cancer, in particular, a number of genes exhibit loss of imprinting with epigenetic silencing of the normally expressed allele. Such genes include, but are not limited to, H19, IGF2, p57^{KIP2} (Matsuoda *et al* (1996) *Proc. Natl. Acad. Sci. USA* 93:3026-3030; and Thompson *et al.* (1996) *Cancer Res. 56*:5723-5727); TSSC3; KvLQT1 (Lee *et al.* (1997) *Nature Genetics 15*:181-185 Rainier *et al.* (1993) *Nature 362:747-749).* However, the methods of the invention are not dependent on knowing the gene involved in the disease.

Restoring a normal pattern of imprinting to cells is involved in a range of diseases affecting many tissues. To date, such diseases include Prader Willi and Angelman syndromes caused by parental-specific disomies (Nicholls et al. (1989) *Nature 342:* 281-285; Knoll et al. (1989) *Am. J.Med. Genet 32:* 285-290; Knoll et al. (1990) *Am. J. Hum. Genet. 47*: 149-155); Mattei et al. (1984) *Hum. Genet 666:* 313-334; Nicholls et al. (1993) *Curr. Opin*. *Genet. Dev. 3*: 445-446; Dittrich et al. (1996) *Nat. Genet. 14*: 163-170; Ledbetter et al. (1995) *Hum. Mol. Genet. 4:* 1757-1764); myotonic dystrophy (DM), Huntington's disease (HD), spinocerebellar ataxia type 1 (SCA 1), fragile X syndrome (FRAXA) and neurofibromatosis types 1 and 2 (Chatkupt et al. (1995) *J. Neurol*. *Sci 130:*1-10); schizophrenia (Ohara et al. (1997) Biol. *Psychiatry 42(9):* 760-766) ; autoimmune disease (Politaev and Selifanova (1994) *Life Sci* 54:1377-1381; Kerzin Storrar et al. (1988) *Neurology 38*: 38-42).

Additionally, loss of normal imprinting patterns are specifically related to oncogenesis and progression of particular cancers (Rainier et al.(1993) *Nature 362*: *747*-749; Feinberg et al. (1993) *Nature Genet. 4:* 110-113; Feinberg et al. (1995) *J. Natl. Cancer Inst. Monographs 17*: 21-26), including Wilm's tumor (Ogawa et al. (1993) *Nature 362:* 749-751); rhabdomyosarcoma (Zhan et al. (1994) *J.Clin. Invest.* 94: 445-448), Ewing's sarcoma (Zhan et *al.* (1995) *Oncogene 11*:2503-2507); germ cell tumors (Van Gurp *et al.* (1994) J. *Natl. Cancer Inst. 86*:1070-1075); choriocarcinoma (Hashimoto *et al.* (1995) *Nature Genet.* 9:109-110); lung cancer (Suzuki et al. (1994) *Nat. Genet. 6*: 332-333); hepatoblastoma (Rainier et al. (1995) *Cancer Res. 55:* 1836-1838); hydatidiform mole (Kajii and Ohama (1977) *Nature* 268:633); parthanogenetic ovarian tetromas (Linder *et al*. (1975) *N. Eng. J. Med.* 292:63-66); paraganglioma or glomus tumor (Van Der May *et al.* (1989) *Lancet* 2:1291-1294); neuroblastoma (Cheng *et al.* (1993) *Nature Genet,* 4:187-190); retinoblastoma (Naumova *et al.* (1994) *Am. J. Hum. Genet. 54*: 274-281); leukemia (Momparler et al. (1997) *Anti-Cancer Drugs 8:* 358-368; Petti et al. (1993) *Leukemia 7(Supp. 1)*: 36-41); hepatocellular cancer and their normal tissue (Takeda *et al.* (1996) *Oncogene 12*:1589-1592); esophageal cancer (Hibi *et al.* (1996) *Cancer Research 56*:480-482); lung cancer (Suzuki *et al*. (1994) *Nature Genetics 6*:332-333 and Kondo *et al*. (1995) *Oncogene 10*:1193-1198); adrenocortical carcinoma, breast cancer, bladder cancer, ovarian cancer, colon cancer, testicular cancer, prostate cancer (Hu *et al*. (1997) *Genomics 46*:9-17, Jarrard *et al*. (1995) *Clin. Cancer Res*. *1*:1471-1478); and Beckwith-Wiedemann syndrome (BWS) (Henry et al. (1991) *Nature 35*: 609-610). Also included are melanomas, leukemias, and cancers of ovary, kidney, and central nervous system origin.

It is recognized that additional cancers and/or diseases will be associated with abnormal imprinting.

LOI can involve activation of the normally silent copy of growth-promoting genes, silencing of the normally transcribed copy of tumor suppressor genes, or both. These changes can involve a switch of one chromosome to the epigenotype of the other chromosome. For example, the maternal chromosome can be switched to the epigenotype of the paternal chromosome.

Pharmacological agents that are capable of altering the imprinting patterns of a cell include agents involved in DNA methylation; such as inhibitors of DNA methyltransferases, topoisomerase II, DNA synthesis, microtubule formation, histone deacetylation, and the like. (Wachsman (1997) *Mutat Res 375 (1)*: 1-8). Specific examples of such agents include 5-azaCdR, Trichostatin A, lankacidin, benzenamine, blue platinum uracil, cyclohexane acetic acid and methyl 13-hydroxy-15-oxo-kaurenoate, etc.

Methods for the determination of the pattern of imprinting are known in the art. It is recognized that the methods may vary depending on the gene to be analyzed. Generally, in methods for assaying allele-specific gene expression, RNA is reverse transcribed with reverse transcriptase, and then PCR is performed with PCR primers that span a site within an exon where that site is polymorphic (i.e., normally variable in the population), and this analysis is performed on an individual that is heterozygous (i.e., informative) for the polymorphism. One then uses any of a number of detection schemes to determine whether one or both alleles is expressed. Methods for the assessment of gene expression, allele specific gene expression, and DNA methylation are encompassed (see Experimental section herein). Additionally, direct approaches to identifying novel imprinted genes include:
positional cloning efforts aimed at identifying imprinted genes near other known imprinted genes (Barlow *et al.* (1991) *Nature 349:84-87);* techniques comparing gene expression in parthenogenetic embryos to that of normal embryos (Kuroiwa *et al.* (1996) *Nat. Genet. 12*:186-190); and restriction landmark genome scanning (Nagai *et al.* (1995) *Biochem. Biophys. Res. Commun. 213*:258-265). See also, Rainier *et al.* (1993) *Nature 362*:747-749; which teaches the assessment of allele-specific expression of IGF2 and H19 by reverse-transcribing RNA and amplifying cDNA by PCR using new primers that permit a single round rather than nested PCR; Matsuoka *et al.* (1996) *Proc. Natl. Acad. Sci USA 93*:3026-3030 which teaches the identification of a transcribed polymorphism in p57^{KIP2}; Thompson *et al.* (1996) *Cancer Research 56*:5723-5727 which teaches determination of mRNA levels by RPA and RT-PCR analysis of allele-specific expression of p57^{KIP2}; and Lee et al. (1997) Nature Genetics 15:181-185 which teaches RT-PCR SSCP analysis of two polymorphic sites.

The methods of the invention are also useful for diagnosis and typing of diseases that are associated with an abnormal pattern of imprinting, and for determining whether such diseases will respond to therapy following the administration of agents that restore a normal pattern of imprinting. For example, the work herein demonstrates that patients with colorectal cancer exhibit LOI in the cancerous tissue, but the normal mucosal cells of these cancer patients also exhibit LOI. Thus, it appears that LOI precedes the development of cancer and can be used to predict the propensity of an individual to the development of cancer or malignant or tumor growth.

Thus, the methods of the invention can be used to determine the propensity of an individual to develop cancer. In those instances where an abnormal pattern has been detected, pharmacological agents can be used to prevent malignant growth or the onset of the disease. Such administration will lead to a restoration of a normal pattern of imprinting and normal monoallelic expression. As more information becomes available regarding associations of specific imprinted loci with particular diseases, the methods of the invention will gain wider applicability in determining the propensity of an individual to such diseases.

### Example 1: Reversal of loss of imprinting in tumor cells.

### Materials and Methods:

Cell Culture and Treatment With 5-azaCdR. JEG-3 human choriocarcinoma and COLO-205 human colon adenocarcinoma cell lines were obtained from ATTC. Cells were cultured in RPMI 1640, 10% fetal calf serum, 5% CO₂, with antibiotics. 10⁴-10⁵ cells in 6 cm dishes were treated with 0.3-10 µM 5-azaCdR for 24 hours. Cultures were washed in Hank's buffered saline solution and grown to confluence. Concentrations of 0.3-3.0 µM caused minimal cytotoxicity (0-30%).

Analysis of Gene Expression. For quantitative analysis of gene expression, total RNA was isolated as described (Chomczynski, P. and Sacchi, N. (1987) *Anal. Biochem.* 162:156-159), and 10 µg was electrophoresed in 1.2% agarose gels containing formaldehyde, and was transferred to Hybond-N+ membranes (Amersham) and hybridized as described with H19 and IGF2 probes (Steenrnan *et al*. (1994) *Nature Genet.* 7:433-439). Hybridization with GAPDH was used as a control for RNA loading. For analysis of allele-specific expression, *0.5* µg RNA was treated with DNase I (Boehringer Mannheim), heated to 95°C for 7 minutes, and RT-PCR was performed as described for both IGF2 and H19 (Rainier *et al.* (1993) *Nature* 362:747-749), although nested PCR using primers HP1 and HP2 (Hashimoto *et al*. (1995) *Nat. Genet.* 9:109-110) was performed for H19 analysis. PCR products were digested with either Apa I (for IGF2 analysis) or Alu I (for H19 analysis) as described (Tadokoro *et al*. (1991) *Nucleic Acids Res.* 19:6967), and electrophoresed on 4% Nusieve-agarose (3:1) gels. Each sample was analyzed in duplicate in the presence and absence of reverse transcriptase to exclude DNA contamination. Gels were blotted and hybridized with a ³²P-labeled internal specific oligonucleotide probe (Rainier *et al.* (1993) *Nature* 362:747-749), and quantified with a PhosphorImager (Molecular Dynamics).

Analysis of DNA Methylation. For quantitative analysis of DNA methylation, genomic DNA was prepared as described (Rainier *et al.* (1993) *Nature* 362:747-749), and Southern analysis of methylation of the H19 promoter CpG island was performed as described (Reik *et al.* (1994) *Hum. Mol. Genet*. 4:2379-2385). Filters were hybridized with the 1.8 kb Pst I fragment of H19, which detects imprint-specific methylation (Steersman *et al.* (1994) *Nature Genet.* 7:433-439). For analysis of allele-specific methylation, 1 µg DNA was digested with BamH I or with BamH I plus Hpa

After digestion, enzymes were inactivated by heating to 100° for 10 min. 50 ng digested DNA was added to the PCR reaction mix in Buffer F (Invitrogen), containing primers HP1 (5'-TACAACC-ACTGCACTACCTG-3') and H3 (5'-TGGAATGCTTGAAGGCTGCT-3') (Hashimoto *et al.* (1995) *Nat. Genet.* 9:109-110). One initial cycle (94°C, 4 min.) was followed by 28 cycles of 94°C, 1 min., 60°C, 1 min., 72° C, 1 min. PCR products (227 bp) were digested with Alu I, electrophoresed on 3% Nusieve/1% agarose gels, transferred to Hybond-N+ membranes (Arnersham), and hybridized with an internal oligonucleotide probe HP2 (5'-TGGCCATGAAGATGGAGTCG-3'), and then quantified with a PhosphorImager (Molecular Dynamics).

### Results:

The purpose of the experiments described here was to determine whether any of the features of loss of imprinting (LOI) in tumor cells was reversible with 5-aza-2'-deoxycytidine (5-azaCdR), a specific inhibitor of DNA methylation. We chose as a model system two tumor cell lines, JEG-3 choriocarcinoma and COLO-205 colorectal carcinoma cells, by screening cell lines for those that fulfilled two criteria: they were heterozygous for transcribed polymorphisms in IGF2 and H19, and thus informative for imprinting studies; and they showed LOI. Cells were treated with 5-azaCdR for 24 hours (approximately 1 cell division) and at concentrations experimentally determined both in our laboratory (Rainier, S. and Feinberg, A.P. (1988) *Proc. Natl. Acad. Sci. USA* 85:6384-6388) and others (Taylor, S.M. and Jones, P.A. (1979) *Cell* 17:771-779) to maximize hypomethylation and minimize cytotoxicity.

We first examined the effect of 5-azaCdR on LOI of IGF2. Tumor cells with LOI show equal biallelic expression of IGF2, in contrast to cells with normal imprinting, which exhibit preferential expression of a specific parental allele (Rainier *et al.* (1993) *Nature* 362:747-749); Ogawa *et al*. (1993b) *Nature* 362:749-751; Rainier *et al*. (1995) *Cancer Res*. 55:1836-1838; Zhan *et al*. (1994) *J. Clin. Invest.* 94:445-448; Suzuki *et al*. (1994) *Nat. Genet.* 6:332-333). As expected, untreated tumor cells, as well as control tumor cells treated with PBS, showed approximately equal expression of the two alleles of IGF2 (Fig. 1A), indicating LOI. In contrast, treatment with increasing doses of 5-azaCdR led to unequal expression of the two IGF2 alleles, with preferential expression of the allele lacking the Apa I polymorphic site (Fig. 1A). Although preferential expression of one allele was partial and not absolute, five repetitions of these experiments always gave the same results, with the predominantly expressed allele always represented by the upper band. Thus, the switch to predominant expression of one allele was not a random effect of 5-azaCdR, but was specific to one allele. Consistent switching by 5-azaCdR from equal biallelic expression to preferential expression of a single parental allele was also observed with a second cancer cell line. Thus, treatment of CQLO-205 colorectal carcinoma cells also showed a switch from biallelic to predominately monoallelic expression of IGF2, and the same allele was preferentially expressed in repeated experiments (data not shown). Thus, 5-azaCdR partially abrogated the pattern of equal biallelic expression of IGF2 in tumor cells with LOI.

We next examined the effect of 5-aza-CdR treatment on overall expression of H19. As we and others had found earlier, LOI of IGF2 is linked in Wilms tumors to down regulation of H19 (Steersman *et al.* (1994) *Nature Genet.* 7:433-439; Moulton *et al.* (1994) *Nature Genet.* 7:440-447). Untreated JEG-3 cells, like Wilms tumors, displayed little to no expression of H19 (Fig. 1B). After treatment with 0.3 µM or 1.0 µM 5-azaCdR, tumor cells showed a substantial increase in H19 expression (Fig. 1B), 30- or 50-fold, respectively, as determined by PhosphorImager analysis. These changes were also reproducible in the second cancer cell line. Thus, COLO-205 cells showed virtually no expression of H19 before treatment. After treatment with 1.0 µM 5-azaCdR, cells showed a 20-fold increase in H19 expression (data not shown). Thus, 5-azaCdR abrogated the epigenetic silencing of H19 in tumor cells with LOI.

We had also observed earlier that LOI of IGF2 in Wilms tumor is associated with biallelic expression of H19 (Rainier *et al*. (1993) *Nature* 362:747-749), despite the relatively low overall quantitative expression of H19 in the tumors. Biallelic expression of H19 is also a characteristic of JEG-3 cells (Hashimoto *et al*. (1995) *Nat. Genet.* 9:109-110. We therefore examined allele-specific expression of H19. We used an RT-PCR assay that reflects allele-specific expression (Ranier *et al*. (1993) *Nature* 362:747-749) but should not be used to quantify total mRNA levels. 5-azaCdR treatment caused a change from biallelic expression to preferential expression of a single H19 allele. This change was even more dramatic than that seen for IGF2, with monoallelic expression of the allele containing the Alu I polymorphic site (Fig. 1C).

We and others showed earlier that LOI in associated with abnormal methylation of the normally unmethylated maternal H19 promoter, to a degree similar to normal methylation of the paternal allele (Steenman *et al*. (1994) *Nature Genet.* 7:433-439; Moulton *et al.* (1994) *Nature Genet.* 7:440-447; Reik *et al*. (1994) *Hum. Mol. Genet.* 3:1297-1301). In order to determine whether treatment with 5-azaCdR altered methylation of this imprint-specific region, cells were digested with Pst I and Sma I, as described (Reik *et al*. (1994) *Hum. Mol. Genet*. 3:1297-1301). Normal cells show approximately 50% methylation, since the paternal allele is methylated and the maternal allele is unmethylated (Steenman *et al.* (1994) *Nature Genet.* 7:433-439; Moulton *et al*. (1994) *Nature Genet.* 7:440-447; Reik *et al*. (1994) *Hum. Mol. Genet*, 3:1297-1301). Cells with LOI show increased methylation, reflecting abnormal methylation of the maternal allele (Steenman *et al.* (1994) *Nature Genet.* 7:433-439; Moulton *et al*. (1994) *Nature Genet.* 7:440-447; Reik *et al.* (1994) *Hum. Mol. Genet.* 3:1297-1301), As expected, control cells treated with PBS showed increased methylation of the H19 CpG island (Fig. 2A). However. after treatment with 5-azaCdR, the H19 CpG island showed a pattern of methylation approaching 50% (Fig. 2A). Like the changes in IGF2 imprinting after 5-azaCdR treatment, these changes in DNA methylation were also reproducible in the second cancer cell line, COLO-205 (data not shown).

Finally, we asked whether the effects of 5-azaCdR were specific to a single allele, as would be expected if these changes were related to changes in genomic imprinting, and not a random effect of 5-azaCdR on both methylated alleles. We PCR-amplified a region of H19 containing a Hpa II site that shows imprint-specific methylation, as well as an Alu I polymorphism that distinguishes the two alleles of the gene. By digesting genomic DNA with Hpa II prior to PCR, we could determine which alleles were methylated before and after treatment of cells with 5-azaCdR. This analysis showed, as expected, that both alleles were methylated in the absence of 5-azaCdR (Fig. 2B). However, after treatment with 5-azaCdR, the allele lacking the Alu I polymorphic site was predominantly amplified (Fig. 2B). Thus, the non-Alu I-containing allele remained methylated, while the Alu 1-containing alle ∼ became unmethylated after 5-azaCdR treatment. Thus, one allele was preferentially affected by 5-azaCdR. Furthermore, the change in IGF2 methylation was not affected by 5-azaCdR (data not shown), again indicating a specific effect of 5-azaCdR on these cells.

In summary, 5-azaCdR restored a pattern of normal genomic imprinting to tumor cells with LOI by each of the following criteria: equal biallelic expression of IGF2 switched to preferential expression of the same allele in five repeated experiments; epigenetic silencing of H19 was reversed; biallelic expression of H19 switched to monoallelic expression of the same allele in all experiments; and hypermethylation of the H19 promoter switched to demethylation of one allele but not the other. These effects on tumor cells with LOI were not random but were specific in each instance to one allele, and they were reproducible both within and between the cell lines. We have shown that 5-azaCdR switched IGF2 from approximately equal expression of maternal and paternal alleles to predominant expression of a specific parental allele in repeated experiments, and caused a switch from biallelic to monoallelic expression of H19 as well as quantitative activation of H19 expression. These effects were not a random response to 5-azaCdR, or else tumor cells would have continued to show equal expression of both alleles of IGF2 and H19. Consistent with the nonrandom effect of 5-azaCdR in these experiment, the loss of DNA methylation of the H19 promoter was specific to one allele. Thus, 5-araCdR treatment confers a normal pattern of genomic imprinting on tumor cells with LOI. This pattern was complete for H19 and partial for IGF2, since in the latter case both alleles were expressed, although the same allele was preferentially expressed in repeated experiments.

These results have three important implications. First, they indicate that abnormal imprinting in tumor cells can be modified pharmacologically. These results are unexpected since previous work with 5-azaCdR would lead one of ordinary skill in the art to conclude that this drug would simply produce hypomethylation of both chromosomes. For example, Eversole-Cire *et al* previously showed that 5-azaCdR treatment of mouse cells with maternal disomy for chromosome 7 causes epigenetic activation of the normally silent maternal IGF2 gene on that chromosome (Eversole-Cire *et al.* (1993) *Mol. Cell. Biol. 13*:4928-4938). Similarly, Hu *et. al* recently found that treatment of normal human brain cells with 5-azaCdR switched IGF2 from a pattern of preferential expression of one allele to equal biallelic expression (Hu *et al.* (1993) *J. Biol. Chem. 271*:18253-18262). In contrast, in the experiments described here using tumor cells with LOI, the pattern of allele-specific expression of IGF2 unexpectedly switched from equal biallelic expression to predominant expression of a single allele. Thus, the results described here are opposite to that taught in the prior art.

Second, these experiments show that some imprint-specific information must still be present in tumor cells with LOI. These results unexpectedly show that the methylation of the H19 promoter affecting expression is independent of the imprint; and, that the imprint is intact but masked by abnormal methylation. Third, this work may provide a novel experimental strategy toward understanding the molecular basis of abnormal imprinting in cancer, since these experiments generate isogenic cell lines that differ in their imprinting of IGF2 and H19. These cell lines can then be tested for alterations at additional target sites, such as p57^{KIP2}, which we have also found to be imprinted (Matsuoka *et al*. (1996) *Proc. Natl. Acad. Sci. USA* 93:3026-3030), or for alterations in transacting factors that may have been activated by 5-azaCdR to mediate these effects. One can use the cells or experiments like those described herein to discover other pharmacologic agents that might influence genomic imprinting.

While the results with 5-azaCdR represent a strategy for drug development, this idea is not limited to that particular compound. Additional drugs have been tested to determine whether or not they show alteration in imprinting. Drugs that show at least some alteration in imprinting and/or cytotoxic effects include lankacidin, benzenamine, blue platinum uracil, cyclohexane acetic acid, methyl 13-hydroxy-15-oxo-kaurenoate, and others. Even drugs which do not show an in vivo effect on imprinting may show such an effect if the drugs are modified biochemically to enhance bioavailability, drug delivery, stability, etc.

### Example 2: Determination of the propensity of an individual to develop cancer or a malignant growth.

### Materials and Methods

DNA and RNA was prepared from tissues from colon cancer patients and normal controls as follows. Colon cancers, normal mucosa from the same patients and normal mucosa from patients who do not have colon cancer but whose colons were removed for other reasons, such as diverticulitis. DNA was extracted by pulverizing the tissue in liquid nitrogen, followed by resuspending in 5ml of TE9 [500mM Tris, 20mM EDTA, 10mM NaCl, pH 9.0]. Samples were incubated overnight at 55°C after addition of proteinase K to 100µg/ml and SDS to a 1 % final concentration. Samples were extracted twice with phenol-chloroform, once with chloroform, and DNA in the samples were ethanol precipitated, washed and resuspended in TE [3mM Tris, 0.2mM EDTA, pH 7.5]. RNA was prepared using the MicroFastTrack mRNA isolation kit from Invitrogen.

### Results

8 informative colorectal cancer were identified with LOI of IGF2. Examination of matched normal tissue revealed that 6 of these 8 patients had LOI of the normal colon mucosal cells. In contrast, of 16 informative patients without colorectal cancer, only 3 of these patients had LOI of the normal nucosa. It is predicted that these patients may have been at risk of developing cancer. In tabular form the results are as shown below.

| | LOI in normal mucosa | No LOI (i.e., normal imprinting) in normal mucosa |
|---|---|---|
| Colorectal cancer with LOI in the cancer tissue | 13 | 3 |
| Noncancer patients | 2 | 6 |

These data are highly significant statistically (p=0.013).

Thus, LOI in normal nucosa is present where there is cancer with LOI, at a significantly higher rate than where there is not cancer. It is predicted that in such "normal" cells with LOI, the LOI would be as reversible by the approach described herein as are cancer cells with LOI. The approach thus, finds use as a chemoprevention strategy for colon cancer with LOI. Likewise, the methods find use in other cancer or tumorigenic growth or other diseases involving LOI.

Although the foregoing invention has been described in some detail by way of illustration and example for purposes of clarity of understanding, it will be obvious that certain changes and modifications may be practiced within the scope of the appended claims.

Many modifications and other embodiments of the invention will come to mind in one skilled in the art to which this invention pertains having the benefit of the teachings presented in the foregoing descriptions and the associated drawings. Therefore, it is to be understood that the invention is not to be limited to the specific embodiments disclosed. Although specific terms are employed, they are used in a generic and descriptive sense only and not for purposes of limitation, and that modifications and embodiments are intended to be included within the scope of the appended claims.

## Claims

1. A process for diagnosing in vitro the propensity of a mammal to develop cancer in a target organ or tissue, said process comprising determining the imprinting pattern of cells from said organ or tissue.

2. The process of Claim 1, wherein said organ or tissue is selected from the group consisting of colon, breast, prostate, liver, lung, bladder, kidney, ovary and testicular.

## Patentansprüche

1. Verfahren zwecks der in-vitro-Diagnose der Neigung eines Saugtieres einen Krebsprozess in einem Zielorgan-oder Gewebe zu entwickeln, wobei das genannte Verfahren die Bestimmung des Abdruckprofiles der aus dem genannten Organ oder Gewebe stammenden Zellen beinhaltet.

2. Verfahren nach Anpruch 1, wobei das genannte Organ oder Gewebe aus der Gruppe bestehend aus Grimmdarm, Brust, Leber, Lungen, Harnblase, Nieren, Eierstock und Hoden ausgewählt wird.

## Revendications

1. Procédé pour le diagnostic *in vitro* de la propension d'un mammifère à développer un cancer dans un organe ou tissu cible, ledit procédé comprenant une détermination du profil d'empreinte des cellules provenant dudit organe ou tissu.

2. Procédé selon la revendication 1, dans laquelle ledit organe ou tissu sélectionné dans le groupe constitué par colon, sein, prostate, foie, poumon, vessie, rein, ovaire et testicule.
